# EUROPEAN PATENT SPECIFICATION

(11) **EP 0 801 897 B1**
(45) Date of publication and mention of the grant of the patent: **03.07.2002**
(21) Application number: 97201133.2
(22) Date of filing: 17.04.1997
(51) Int. Cl.: A01N 59/00, A23L 3/358, C02F 1/72

(54) **Composition for disinfecting raw materials, products and production means, method for its preparation, and disinfection method**
Zusammensetzung zum Desinfizieren von Rohstoffen, Produkten und Produktionsmitteln, Verfahren zu ihrer Herstellung, und Desinfizierungsverfahren
Composition pour la désinfection de matières premières, de produits et de moyens de production, procédé de sa préparation, et procédé de désinfection

(30) Priority: 17.04.1996 NL 1002887; 11.06.1996 NL 1003316
(43) Date of publication of application: 22.10.1997
(73) Proprietor: UniProx B.V., 1171 VL Badhoevedorp (NL)
(72) Inventor: van Os, Jan, 1312 SW Almere (NL)
(74) Representative: Van Breda, Jacobus

(56) References cited:
- EP-A- 0 423 922
- WO-A-93/02973
- WO-A-93/04595
- DATABASE WPI Section Ch, Week 8944 Derwent Publications Ltd., London, GB; Class D25, AN 89-321432 XP002020108 & JP 01 240 600 A (SEIWA KOGYO KK) , 26 September 1989
- DATABASE WPI Section Ch, Week 9432 Derwent Publications Ltd., London, GB; Class D25, AN 94-260811 XP002020109 & JP 06 192 692 A (KAO CORP) , 12 July 1994

## Description

The invention relates to a composition for disinfecting raw materials, products, water or production means, in particular in the food processing industry, which composition is prepared from hydrogen peroxide and glycerol. In addition, the invention relates to a method for preparing this composition and a method for disinfecting raw materials, products, water and/or production means, in which the composition mentioned is utilized.

Such a composition and such a decontamination method are described in European patent application 0 603 329.

In that publication a composition is described having hydrogen peroxide and glycerol therein, which composition can be utilized for disinfecting a variety of materials. In addition, this European patent application describes apparatuses for carrying out that method, which apparatuses, for that matter, can also be used for the present invention.

The contamination of raw materials and processed products with harmful biological material is more and more regarded as objectionable in view of public health and the environment. Especially materials and equipment that are used in the food processing industry must satisfy increasingly stringent requirements regarding their bacteriological condition. The problem is especially urgent in, for instance, processes in the food processing industry where conveyors and the like are employed, at room temperature, because there the containment and control of germs and other harmful constituents is difficult.

It is known to improve the storage properties of foods by adding chemicals. Thus, according to U.S. Patent 2,053,740, hydrogen peroxide can be added to milk and cream, or substances that produce hydrogen peroxide under the action of enzymes can be added to foods (German patent specification 2.420.135). Also, monoalcohols and polyalcohols have been used for the treatment of foods. (EP-A-169 927, DE-A-2 119 351, GB-A-1 273 938 and GB-A-1 375 704).

In European patent application EP-A 0 460 962, a method is described in which vegetable foods are sprayed with an aqueous solution which can contain up to 0.025% of an organic peracid. Then follows a treatment with aqueous thiosulfate. The object of this European patent application is to perform a disinfection and to improve product appearance.

DE-A-3 806 953 describes a solution for cleansing contact lenses. Present in this solution is a disinfectant in the form of hydrogen peroxide. This disinfectant can be combined with a borate, glycerol, aminoacetic acid or an acetate or phosphate for obtaining an isotonic solution.

Also widely known is the addition of sulfite to keep the contamination of foods at an acceptable level. For cleaning and disinfecting production equipment, often use is made of chlorine. It is noted that the use of chemicals entails the risk of metering too high amounts, so that sometimes carcinogenic or allergenic effects can arise. The use of chlorine can also lead to corrosion.of metal-containing. equipment, which in itself can constitute a source of contamination with bacteria. A further disadvantage of the chemical agents used heretofore is that the excess thereof is carried along with cleaning water and thus can be harmful to the environment.

EP-A-0 423 922 describes an antimicrobial composition containing H₂O₂, lactic acid and a sequestrant.

Database, WPI, Section Ch, week 9432, AN: 94-260811 relates to a liquid bleach detergent composition comprising hydrogen peroxide, surfactants and a mixture of glycolic and lactic acid, useful as toilet cleaner.

A solution to such problems has been sought in working in an aseptic space or in the use of waste water purification. Such water purification plants are mostly designed on the basis of the average water quality, the bacteriological load and the effluent standards. Failures in the production then often lead to overloading of the plant, so that chemicals must be resorted to again to satisfy product requirements. All this leads to a sometimes unacceptable cost increase and environmental harm.

Accordingly, there remains a great need for a cheap and simple method for disinfecting raw materials, products and production means, as well as process water of such a production.

According to the present invention, it has now been found that the composition should satisfy claim 1.

In particular, thus a composition is obtained which exhibits a considerably faster disinfectant action, without the effective duration of that composition being shortened.

While a composition according to EP-A-0 603 329 scores a value of 15 minutes in the European Suspension Test (EST-test; 3x5 test), the composition according to the invention scores 6-7 minutes.

The composition according to the invention is prepared from hydrogen peroxide, glycerol and glycolic acid.

Another important advantage of the composition of the present invention over that described in EP-A-0 603 329 is that it exhibits a far better virus-, fungus- and spore-killing activity. For that matter, in EP-A-0 603 329, preferably an organic acid is used as stabilizer. Of the organic acids specifically mentioned, viz. acetic acid, lactic acid, benzoic acid, hexanoic acid, valeric acid, propionic acid and higher fatty acids, it is preferred to use valeric acid as stabilizer. This acid is preferably present in an amount of 1-5% by weight with respect to the hydrogen peroxide. No mention is made of any advantage in the speed of action. Moreover, organic peracids are formed from the stabilizers mentioned, which react considerably more aggressively than compounds from the present composition. The organic acids specifically mentioned further form toxins from microorganisms to be killed off.

In addition, it is pointed out that glycolic acid cannot simply be added straightforwardly. This would lead to vehement reactions.

Accordingly, the present invention also relates to a method for preparing the composition.

The method for preparing this composition comprises mixing a solution of glycerol with a solution of glycolic acid, and dropwise adding the mixture obtained to a solution of hydrogen peroxide under such conditions that the solution of hydrogen peroxide maintains a temperature below 7°C and preferably below 4°C.

Essentially, in this method after each addition of a hydrogen peroxide solution to a mixture of glycolic acid and glycerol, quenching follows. This means that with stirring, and preferably with vigorous stirring, and with cooling, in each case such an aliquot of peroxide solution is added, that the temperatures before and after addition differ from each other by not more than 1°C and preferably less than 1/2°C.

The invention further relates to a method for decontaminating raw materials, products, water and/or production means, in which the composition according to the invention or the product according to the method just mentioned is contacted with the object to be decontaminated.

More in detail, the composition according to the invention can make a major contribution to an improvement of the heat transfer in closed cooling and circulation systems, because this composition keeps the systems free from. accretions of organic, inorganic and bacteriological pollutions. In addition, it contributes to the removal or termination of already existing pollutions in those systems.

The use of the present product in water transport systems, for instance in glasshouse horticulture, will prevent algal growth and accretions of organic pollutions therein, while the germ-free water has a positive influence on the storage life of the products (*inter alia* tomatoes, paprikas, and the like) which are transported using the water transport system.

In closed cooling water/feed water systems, for instance in glasshouse horticulture, this product will evidence better cleaning than the agents now commonly used; it also prevents scaling up in drip systems.

When the composition according to the invention is used, it is possible, in any system where inhibitors are metered to prevent corrosion, to switch to the product mentioned, because it inhibits corrosion and does not leave any residues which promote clogging and/or fouling. Further, the water will not contain any toxins coming from the dosed product, so that the process water from a system is discharged to the surface water.

Also, the composition can be used well in open as well as closed industrial cooling water systems.

The composition according to the invention can also be used in cooling air for which the organic and bacteriological purity is a prerequisite, such as cooling air in slaughterhouses and bakeries where contamination of polluted air with the food will contribute to an accelerated decay of the product. Another application of the product according to the invention lies on fishing boats where, for instance, shrimps and cockles are boiled on board and cooling is carried out with outboard water. This cooling/process water is to a large extent responsible for the high germ count of those products. Now, the composition according to the invention provides a fast organic and bacteriological cleaning of the cooling water. From the cooling/process water, no or substantially no after- or re-contamination. occurs on the boiled product. In seawater, moreover, a component is present which seems to have a catalyzing action on the composition according to the invention.

The washing of fish and product boxes in the food processing industry can therefore be carried out equally well using the composition according to the invention. For a very long time these products are safeguarded from bacteriological contaminations.

The composition according to the invention lends itself in particular for misting in stables and atomization in product spaces, because it gives a faster action than the composition according to EP-A-0 603 329. Moreover, it is far safer for the surroundings than other known agents. This is chiefly due to the environmental aspects which are inherent to this composition.

Thus, the composition according to the invention is generally highly suitable and hence very useful for "cleaning in place" (C.I.P.) in breweries, beverages, dairy industries, and milk production equipment and as additive in cleaning water for apparatus as used in the food processing industry.

A water circulation system in which the composition according to the invention is used will need to be refreshed less often, since the composition according to the invention conditions the water for a long time.

When using these products, for that matter, it is desirable to mount a filter in the circulation system to separate solid pollutions which have ended up in the water.

Owing to the water remaining, or becoming, clean in contact with the composition according to the invention, a reduction of 60-70% of the required amount of water can be realized.

The composition according to the invention can be contacted with unprocessed foods without any objection.

Any decomposition products of the composition according to the invention are substances that are not foreign to the body or toxic.

For cleaning and preserving hatching eggs and shell eggs, the composition according to the invention will also have a faster action without being aggressive. The product then yields a prolonged sustaining and nursing action on the shell of the egg. This is attributed to the activating action which derives from the glycolic acid in the solution and hence has a potentiating action on organic and bacteriological pollutions.

The product suitably lends itself for preserving citrus fruits, which must be protected for a prolonged time against fungoid growth and/or bacterial after-/re-contamination during storage and transport.

Preferably, the means or materials are disinfected by atomizing the above-mentioned solution under excess pressure in the space where they are located. This manner of disinfection/conservation works very efficiently with a very low consumption of disinfecting agent, because disinfection proceeds in the gas phase. The food thus sustains no damage from the disinfectant.

When disinfecting foods, it is preferred to use such an amount of the solution that per kg of food 0.1-1 g of hydrogen peroxide is used.

Disinfection in the above-mentioned way takes place in particular at a temperature between 5 and 70°C for about 1 minute to about 1/2 hour, depending on the treatment temperature and the concentration of the solution in the space.

As stated, the composition according to the invention is prepared at least from hydrogen peroxide, glycerol and glycolic acid.

For instance, and preferably, the composition can be prepared from:

| | |
|---|---|
| Hydrogen peroxide | 50% stab: |
| Glycerol (1.26) | clinically pure |
| Glycolic acid | 57% |

These compounds are employed in an aqueous solution. Preferably, demineralized water is used as solvent system.

Glycolic acid, before being added to hydrogen peroxide, is mixed with the glycerol. This is necessary to arrive at a properly working composition. This preferably occurs in the following way.

The glycolic acid is added to the glycerol, to prevent explosive reactions that would arise upon addition of glycolic acid to a mixture of hydrogen peroxide and glycerol.

The ratio of glycerol to glycolic acid is preferably between 5:1 and 25:1.

The glycerol solution, for instance glycerol (1.26), can, in the mixture with, for instance, 57% glycolic acid, be added to (for instance 50%-) hydrogen peroxide in motion.

When these components are being put together, it is very important that the solution is immediately cooled back to 7°C or less, preferably 4°C or less. The dropwise addition preferably may not give any temperature rise in excess of 1°C, preferably less than 0.5°C, because this adversely affects the quality of the composition. The whole must remain in the mixing vessel for so long, for instance at least 20 minutes, to ensure a homogeneous mixture, while preferably maintaining a temperature of less than 4°C for at least 10 minutes.

In a preferred embodiment all this takes place in an on-line mixer.

Upon storage and transport the thus obtained solution remains stable for a prolonged time, for instance for 3 weeks up to six months.

Storage should be done in a cool place. The composition is then preferably separated from oxidizing products and organic material, such as wood, textile, paper and alkaline agents.

In brine or seawater the solution is found not to decompose appreciably due to the chlorides, such as AgCl, that are present therein. These compounds even seem to have a catalyzing action.

This is important because some applications for which the composition according to the invention is useful entail contact with brine/seawater of 20 degrees Brix or higher.

The concentration of glycolic acid in the composition according to the invention depends on the amount of glycerol and hydrogen peroxide. Preferably, however, not more than 25 grams of glycolic acid per kilogram of composition is used.

In a preferred composition 125 grams of glycerol per kilogram of composition is used, and 5 to 25 grams of glycolic acid per kilogram of composition will be present. All this can depend on the application and the extent of pollution to be decontaminated.

Detailed ratios of the components can be as follows.

Glycerol having dissolved glycolic acid therein is present in the ratios of:

| Concentration of glycerol (g/kg) | 100 | 125 | 150 | 175 |
|---|---|---|---|---|
| Ratio of glycolic acid:glycerol | 1:20 | 1:25 | 1:30 | 1:35 |
| preferably | 1:10 | 1:15.5 | 1:15 | 1:17.5 |
| more preferably | 1:6.5 | 1:8.5 | 1:10 | 1:11.7 |
| preferred | 1:5 | 1:6.25 | 1:6.5 | 1:6.75 |
| and most preferred | 1:4 | 1:5 | 1:6 | 1:7 |

Surprisingly, the addition of glycolic acid which is dissolved in the glycerol and hydrogen peroxide is found to have such an action as to give the composition a faster action, while retaining a prolonged protection from after- and re-contamination of the products or air or water streams to be treated.

It is assumed that this effect is based on Pauling and de-pole action. This makes the composition highly important in warmer surroundings, as in glasshouse horticulture and in regions with relatively high temperatures.

Surprising about the present invention is that when hydrogenperoxide is mixed with glycerol and glycolic acid dissolved therein, all in a solution of preferably demineralized water, and this solution is then atomized on or around the unprocessed or processed food or apparatus (air conditioning), an effective preservation takes place, whereby basically all bacteria, yeasts, fungi, germs and the like are killed off.

The action in respect of fungi on plants and in spaces where fungoid growth is more of a rule than an exception, is to be rated "considerable", in particular in comparison with the composition according to EP-A-0 603 329.

The preserving agent (decontaminant) according to the invention is characterized in that it consists of a solution of hydrogen peroxide and glycerol with glycolic acid dissolved therein, preferably in demineralized water.

The invention also relates to a method for decontaminating raw materials, products, water and/or production means, including space, in which the composition according to the invention or the products prepared according to the present method are contacted with the means to be decontaminated. This can be done by contacting the means with the solution in any known way. Disinfection with the composition according to the invention can take place in varying ways, for instance through spraying, irrigation, immersion, rinsing, and the like. Preferably, the composition is atomized. Thus the atomization can occur according to the principle of an air humidifier and be used for the storage of foods, for instance in cooling or deepfreeze spaces for the storage of foods such as potatoes, or without special cooling facilities. When the composition according to the invention is atomized in the space around the materials to be disinfected, this preferably occurs in such a manner that the concentration of hydrogen peroxide in the air is 0.001 to 0.01% by volume. The treatment time depends on the concentration of the mixture in the atmosphere and on the temperature. Thus, at a concentration of between 0.001 and 0.005% by volume and a temperature of 5 to 25°C the treatment time will typically be between 1 and 5 minutes. When disinfecting with the mixture as a solution with a concentration of 0.2-0.3% by weight of hydrogen peroxide and a temperature of 50 to 60°C, the treatment time will typically be between 3 and 20 minutes.

The mixture of hydrogen peroxide, glycerol and glycolic acid according to the invention is a clear and virtually odorless liquid which is weakly acid in reaction. The mixture has a broad disinfecting action and eliminates *inter alia* bacteria, fungi, algae and viruses. The composition counteracts slime formation and does not give any foam formation. Disinfection of foods with the mixture does not give any discolorations or changes in odor and taste.

The mixture can be used in the entire food industry, in the production, processing and storage of foods. The apparatus described in EP-A-0 603 329 can also be used in both large and small production units. This apparatus comprises a conveying means for the material or product to be treated and at least one disinfection chamber through which the material or product can be passed. The disinfection chamber is provided with means for atomization and/or spraying of a liquid disinfecting agent, as well as with means for metering, pumping, and removing the disinfectant.

## Claims

1. A composition for decontaminating raw materials, products, water and/or production means, which composition is prepared from at least hydrogen peroxide, glycerol and glycolic acid.

2. A composition according to claim 1, wherein the ratio of glycerol to glycolic acid lies between 5:1 and 25:1.

3. A method for preparing the composition according to any one of claims 1-2, comprising mixing a solution of glycerol and a solution of glycolic acid, and dropwise adding the mixture obtained to a solution of hydrogen peroxide under such conditions that the solution of hydrogen peroxide maintains a temperature below 7°C and preferably below 4°C.

4. A method according to claim 3, wherein the conditions are set in such a manner that the temperatures before and after adding a charge of hydrogen peroxide solution differ from each other by not more than 1°C and preferably less than 1/2°C.

5. A method according to claim 3 or claim 4, wherein the mixing of the solutions takes place in an on-line mixer.

6. A method according to any one of claims 3-5, wherein the whole is post-mixed for so long that the final composition is homogeneous.

7. A method according to claim 6, wherein the first phase of the after-mixing takes place at a temperature below 7°C, preferably below 4°C.

8. A method for decontaminating raw materials, products, water and/or production means, comprising contacting the composition according to any one of claims 1-2 or obtained by the method according to any one of claims 3-7 with the means to be decontaminated.

## Patentansprüche

1. Eine Zusammensetzung zum Dekontaminieren von Rohmaterialien, Produkten, Wasser und/oder Produktionseinrichtungen, wobei die Zusammensetzung zumindest aus Hydrogenperoxid, Glycerol und Glykolsäure hergestellt ist.

2. Eine Zusammensetzung gemäß Anspruch 1, bei der das Verhältnis von Glycerol zu Glykolsäure zwischen 5:1 und 25:1 liegt.

3. Ein Verfahren zum Herstellen der Zusammensetzung gemäß einem der Ansprüche 1 bis 2, das das Mischen einer Lösung aus Glycerol und einer Lösung aus Glykolsäure umfaßt, und das tropfenweise Hinzufügen der erhaltenen Mischung zu einer Lösung aus Hydrogenperoxid unter solchen Bedingungen, daß die Lösung aus Hydrogenperoxid eine Temperatur unterhalb 7°C und vorzugsweise unterhalb 4°C beibehält.

4. Ein Verfahren gemäß Anspruch 3, bei dem die Bedingungen auf solche Weise eingestellt sind, daß sich die Temperaturen vor und nach dem Hinzufügen einer Ladung von Hydrogenperoxidlösung um nicht mehr als 1°C und vorzugsweise um weniger als 1/2°C voneinander unterscheiden.

5. Ein Verfahren gemäß Anspruch 3 oder Anspruch 4, bei dem das Mischen der Lösungen in einem Prozeßmischer stattfindet.

6. Ein Verfahren gemäß einem der Ansprüche 3 bis 5, bei dem das Ganze so lange nachgemischt wird, bis die Endzusammensetzung homogen ist.

7. Ein Verfahren gemäß Anspruch 6, bei dem die erste Phase des Nachmischens bei einer Temperatur unterhalb von 7°C, vorzugsweise unterhalb von 4°C stattfindet.

8. Ein Verfahren zum Dekontaminieren von Rohmaterialien, Produkten, Wasser und/oder Produktionseinrichtungen, das das Kontaktieren der Zusammensetzung gemäß einem der Ansprüche 1 bis 2, die durch das Verfahren gemäß einem der Ansprüche 3 bis 7 erhalten wird, mit der zu dekontaminierenden Einrichtung umfaßt.

## Revendications

1. Composition pour la désinfection de matières premières, de produits, d'eau et/ou de moyens de production, ladite composition étant préparée à partir d'au moins du peroxyde d'hydrogène, du glycérol et de l'acide glycolique.

2. Composition selon la revendication 1, dans laquelle le rapport glycérol/acide glycolique se situe entre 5:1 et 25:1.

3. Procédé de préparation de la composition selon l'une quelconque des revendications 1 à 2, comprenant le mélange d'une solution de glycérol et d'une solution d'acide glycolique, et l'ajout goutte à goutte du mélange obtenu dans une solution de peroxyde d'hydrogène, dans des conditions telles que la solution de peroxyde d'hydrogène reste à une température inférieure à 7°C et de préférence inférieure à 4°C.

4. Procédé selon la revendication 3, dans lequel les conditions sont déterminées de telle sorte que les températures avant et après l'ajout d'une charge de solution de peroxyde d'hydrogène diffèrent l'une de l'autre d'au plus 1°C et de préférence de moins de 1/2°C.

5. Procédé selon la revendication 3 ou la revendication 4, dans lequel le mélange des solutions se produit dans un mélangeur en ligne.

6. Procédé selon l'une quelconque des revendications 3 à 5, dans lequel l'ensemble est soumis à un post-mélange jusqu'à ce que la composition finale soit homogène.

7. Procédé selon la revendication 6, dans lequel la première phase du post-mélange se déroule à une température inférieure à 7°C, de préférence inférieure à 4°C.

8. Procédé pour la désinfection de matières premières, de produits, d'eau et/ou de moyens de production, comprenant la mise en contact d'une composition selon l'une quelconque des revendications 1 à 2, ou obtenue par le procédé selon l'une quelconque des revendications 3 à 7, avec les moyens devant être désinfectés.
